# EUROPEAN PATENT APPLICATION

(11) **EP 2 548 971 A1**
(43) Date of publication of application: **23.01.2013**
(21) Application number: 11174434.8
(22) Date of filing: 19.07.2011
(51) Int. Cl.: C12Q 1/68

(54) **CHAC1 transcript variants as marker for breast cancer**

(71) Applicant: Oncotyrol Center for Personalized Cancer Medicine GmbH, 6020 Innsbruck (AT)
(72) Inventor: Fiegl, Heidelinde, A-6083 Ellbögen (AT); Berger, Regina, A-6091 Götzens (AT)
(74) Representative: Vögele, Andreas

(57) **Abstract**

A method to determine the survival outcome of a breast cancer patient or to determine prognosis of a breast cancer patient, wherein the method comprises assaying a sample of breast cancer cells of the breast cancer patient for the expression level of CHAC1 transcript variants 1 and/or 2.

SEQUENCE LISTING

<110>  ONCOTYROL ˜ Center for Personalized  Cancer Medicine GmbH

<120>  CHAC1 transcript variants as marker for breast cancer

<130>  TY10169

<160>  6

<170>  PatentIn version 3.5

<210>  1
<211>  16
<212>  DNA
<213>  Artificial Sequence

<220>
<223>  PCR Primer

<400>  1
atgcctggcc gtgtgg                                                        16


<210>  2
<211>  19
<212>  DNA
<213>  Artificial Sequence

<220>
<223>  PCR Primer

<400>  2
   gcttacctgc tccccttgc                                                      19


<210>  3
<211>  26
<212>  DNA
<213>  Artificial Sequence

<220>
<223>  PCR Primer

<400>  3
cagccctcat gatcttcaag gagcgt                                             26


<210>  4
<211>  18
<212>  DNA
<213>  Artificial Sequence

<220>
<223>  PCR Primer

<400>  4
ggttctgctc cccttgca                                                      18


<210>  5
<211>  19
<212>  DNA
<213>  Artificial Sequence

<220>
<223>  PCR Primer

<400>  5
cgtgtggtga cgctccttg                                                    19


<210>  6
<211>  20
<212>  DNA
<213>  Artificial Sequence

<220>
<223>  PCR Primer

<400>  6
cccaagtgca gccctcatga                                                   20

## Description

The invention relates to assays comprising oligonucleotides, capable of hybridizing to mRNA of CHAC1 transcripts. The invention also relates to a method to determine the survival outcome of a breast cancer patient or to determine prognosis of a breast cancer patient. Further, the invention relates to a method to determine therapeutic treatment for a breast cancer patient based upon said patient's expected survival.

Breast cancer is by far the most frequent cancer among women with an estimated 1.38 million new cancer cases worldwide diagnosed in 2008 (23 % of all cancers), and ranks second overall (10.9 % of all cancers) (1). For women between ages 50 and 55 breast cancer is the leading cause of death. Based on cancer rates from 1995 through 1997, a report from the National Cancer Institute (NCI) estimates that about one in eight women in the United States (approximately 12.8 percent) will develop breast cancer during her lifetime (2).

Although the presence or absence of metastatic involvement in the axillary lymph nodes is the most powerful prognostic factor available for patients with primary breast cancer (3), it is only an indirect measure reflecting the tendency of the tumour to spread.

Extensive research has been conducted in the field of early detection, treatment and prevention of breast cancer. Precancerous or cancerous ductal epithelial cells are analyzed, for example, for cell morphology, for biomarkers (protein markers, nucleic acid markers, biochemical markers), and for chromosomal abnormalities. A variety of molecular alterations have been reported in breast cancer. These molecular alterations include presence/absence of estrogen and progesterone steroid hormone receptors, HER-2 expression/amplification (4), Ki-67, and prognostic markers including oncogenes, tumour suppressor genes, and angiogenesis markers.

The unfolded protein response (UPR) pathway is activated in a range of human solid tumours including breast cancer (5). It was shown that the UPR activation may contribute to breast cancer chemoresistance and interact with oestrogen response elements (6).

Recently CHAC1 (cation transport regulator-like protein 1 or MGC4504) has been identified as a component of the UPR pathway which itself responds to endoplasmic reticulum stress (7). In this context, it has been shown, that hypoxia enhances the metastatic potential of many tumours and that glucose deprivation is linked to higher tumour grade in breast cancer (8). Endoplasmic reticulum stress initiated by the tumour microenvironment and the activation of the UPR is proposed to lead to different disease outcomes, like apoptosis and tumour resolution, tumour dormancy, tumour growth and disease progression or altered chemotherapeutic sensitivity (9). Two alternatively spliced transcript-variants of this molecule are described until now. Transcript-variant 1 [GenBank: NM_024111.3] represents the longer transcript and encodes the longer "Isoform a". Transcript-variant 2 [GenBank: NM_001142776.1] lacks an alternate in-frame segment compared to variant 1, resulting in a shorter protein ("Isoform b") compared to "Isoform a".

US 2005/0100933 A1 (Erlander et al.) investigated the presence of certain cancer markers amongst which MGC4504.

### Summary of the Invention

Determining the presence or absence of metastatic involvement in the axillary lymph nodes for determining prognosis of the breast cancer recurrence is only an indirect measure. The prior art thus suffers from direct measurement routes for determining prognosis and treatment of breast cancer patients. US 2005/0100933 A1 (Erlander et al.) - as will be discussed in more detail below - mention a wide variety of markers possibly linked with breast cancer. Yet, the statistical results of Erlander et al. for some of these markers are not significant for the prediction of breast cancer recurrence and therefore poor markers.

It is therefore an object of the present invention to provide a direct and specific way to determine the risk of breast cancer recurrence of a patient.

This object is solved by the means provided in the independent claims.

According to the invention an assay to determine the amount of CHAC1 transcript present in a sample is presented which is characterized in the use of oligonucleotides, capable of hybridizing to mRNA of CHAC1 transcript variant 1 or CHAC1 transcript variant 2.

Further, a method to determine the survival outcome of a breast cancer patient or to determine prognosis of a breast cancer patient is presented which is characterized in that the method comprises the analysis of breast cancer cell samples of the breast cancer patient for the expression level of CHAC1 transcript variants 1 and/or 2.

Next, a method to determine therapeutic treatment for a breast cancer patient based upon said patient's expected survival is presented which is characterized in that the method comprises determining a survival outcome for said patient by analyzing a sample of breast cancer cells from said patient for the mRNA expression level of CHAC1 transcript variants 1 and/or 2 and selecting the appropriate treatment for a patient with such a survival outcome.

Next, a method to determine the expression level of CHAC1 transcript variants 1 and/or 2 present in a sample of breast cancer patient is presented by measuring the amount of the mRNA present in the breast cancer cells.

Finally, the invention relates to an oligonucleotide selected from the group SEQ ID NOS: 1, 2, 3, 4, 5, and 6.

The invention provides for the use of CHAC1 transcript variants 1 and/or 2 with clinical relevance to breast cancer. In detail, the invention provides evidence of a correlation of CHAC1 transcript variant 1 and/or 2 mRNA expression with patient overall survival and breast cancer recurrence. The mRNA expression may also be used in the study and/or determination of prognosis of a patient. When used for prognosis, the CHAC1 transcript variant 1 and/or 2 mRNA expression may be used to determine the treatment of breast cancer based upon the likelihood of life expectancy and recurrence.

The invention provides for the use of CHAC1 transcript variant 1 and/or 2 mRNA expression which correlates with (and therefore, capable of discriminating between) patients with good or poor survival outcomes.

The invention also provides for the use of CHAC1 transcript variants 1 and/or 2 mRNA expression which correlates with the distant recurrence of breast cancer in terms of metastases. CHAC1 transcript variants 1 and/or 2 mRNA expression is able to distinguish patients with breast cancer into those with good or poor survival outcomes.

The present invention provides an objective method for the identification of patients with breast cancer as likely to have a good or poor survival outcome by analyzing CHAC1 transcript variants 1 and/or 2 mRNA expression. Therefore, instead of a subjective interpretation to determine the prognosis and/or treatment of breast cancer patients, the present invention provides objective CHAC1 transcript variants 1 and/or 2 mRNA expression levels, which can be used with subjective criteria to provide a more accurate assessment of breast cancer patient outcomes, including survival and the recurrence of breast cancer. Thus, CHAC1 transcript variants 1 and/or 2 mRNA expression analysis of the invention provides a means to determine breast cancer prognosis.

Elevated expression of each one of the two CHAC1 transcript variants is correlated with increased likelihood of tumour recurrence and decreased patient survival in a multivariate analysis.

CHAC1 transcript variants 1 and/or 2 mRNA expression analysis may be used to analyze a sample from a breast cancer patient to predict the outcome of the breast cancer patient. Such assays may be used as part of a method to determine the therapeutic treatment for said breast cancer patient based upon the breast cancer outcome identified.

In another aspect, the invention provides methodological means for detecting CHAC1 transcript variants 1 and/or 2 mRNA expression.

In US 2005/0100933 A1 (Erlander et al.) a variety of breast cancer markers is listed. In Table 6 Erlander et al. mention MGC4504 among 141 genes which correlate with breast cancer recurrence. MGC4504 is listed in 134th place in this gene-list which is ranked according the statistical significance. These 141 genes were identified from a starting gene pool of 180 genes, wherein the 141 genes had expression levels that correlated with the absence or presence of breast cancer recurrence. But approximately 94 % of the genes described in this table do not yield a level of significance that would remain significant after multiple test adjustment. In statistics, the multiple comparisons or multiple testing problems occur when one considers a set of statistical inferences simultaneously. Multiple test correction usually is applied to keep the (overall) probability of the Type I error below a reasonable level (usually < 0.05). If no multiple test correction is applied, it becomes more likely that the groups being compared will appear to differ in terms of at least one attribute. If the number of statistical tests are too high, the probability to detect a difference (exactly spoken: the probability of facing a Type I error) converges to 1. The so-called Bonferroni correction is an example of a statistical method used to address the problem of multiple comparisons. If in an analysis/ experiment *n* dependent or independent hypotheses are tested on a set of data, it is likely that the results have occurred by chance. Therefore the significance level must be scaled down by dividing it by "*n*", the number of different hypotheses. Erlander et al. analyzed initially 180 genes. Therefore only p-values less than 0.00027 (that is the significance level of 0.05 divided by 180) are statistically significant. After this Bonferroni correction only the first eight listed genes would remain significant. The residual 133 genes including MGC4504 which is listed in 134th place with a p-value of 0.0117 would not remain significant after applying the multiple test correction. Also in our univariate survival analysis (overall and distant disease free survival) we observed that the mRNA expression analysis of CHAC1 could not predict the survival of the breast cancer patients. Only the mRNA expression of transcript variant 2 of CHAC1 coding for the Isoform b was a suitable prognostic marker in univariate survival analysis. In the multivariate analysis also transcript variant 1 coding for the Isoform "a" was a suitable prognostic marker.

Definitions of terms as used herein:
- A transcript variant refers to the fact that many genes have more than one transcript due to alternative splicing.
- The terms "correlate" or "correlation" refer to an association between expression of the described CHAC1 transcript variants and a physiologic state of breast cancer or outcome.
- An "oligonucleotide" is a molecule usually composed of 50, preferably 25, or fewer nucleotides; used as a DNA synthesis primer.
- A "breast tissue sample" refers to a sample of breast tissue from an individual suspected of being afflicted with breast cancer. Such samples are primary isolates (in contrast to cultured cells) and may be collected by an invasive method, including, but not limited to, surgical biopsy.
- Overall survival is an indication of the proportion of people within a group who are expected to be alive after a specified time. It takes into account death due to any cause - both related and unrelated to the cancer in question.
- Distant disease free survival measures the proportion of people among those treated for a cancer whose disease will remain stable (without signs of progression) at a specified time after treatment. It takes into account the occurrence of distant metastases or death due to any cause - both related and unrelated to the cancer in question.
- Q-PCR is based on the PCR and which is used to amplify and simultaneously quantify a targeted DNA molecule.

With reference to the figures and the following description of specific embodiments further details and advantages of the invention are shown.

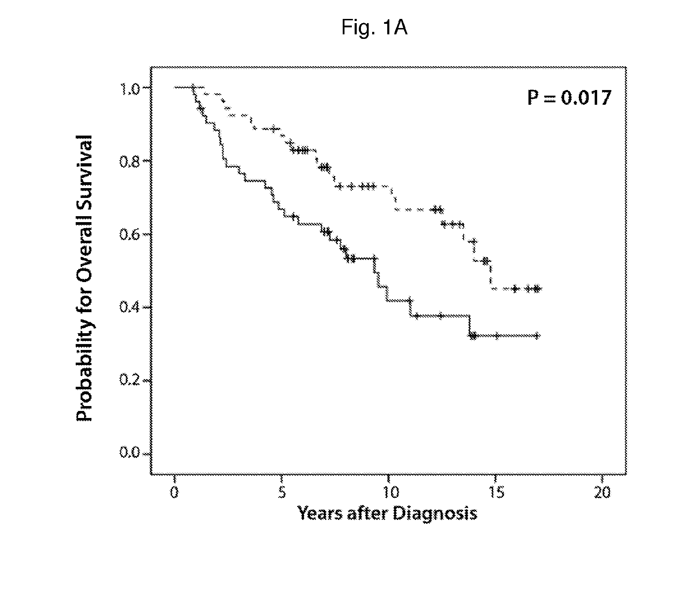

- Fig. 1A: shows the overall survival analysis in 106 breast cancer patients and CHAC1 transcript variant 2 mRNA-expression.

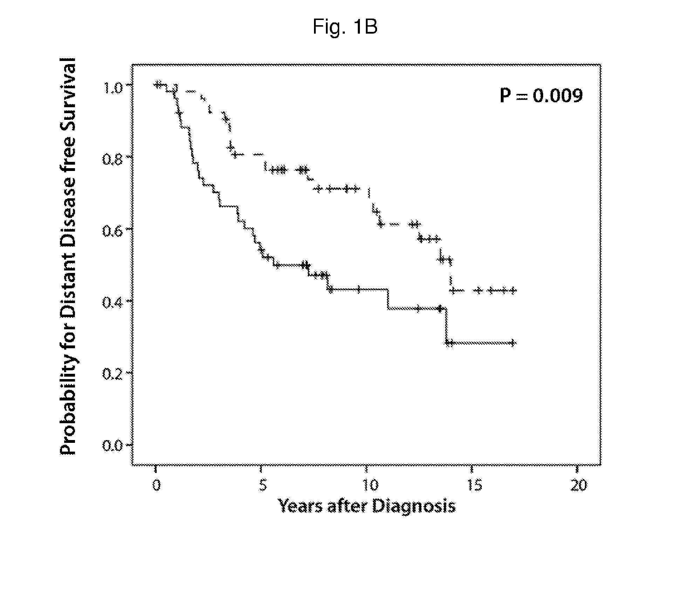

- Fig. 1B: shows the distant disease free survival analysis in 106 breast cancer patients and CHAC1 transcript variant 2 mRNA-expression.

The present invention shows that both CHAC1 transcript variant 1 and 2 mRNA expression are correlated with breast cancer recurrence, yet CHAC1 transcript variant 2 mRNA expression which discriminate between (or is correlated with) breast cancer survival and recurrence outcomes in a breast cancer patient is highly significant in univariate and multivariate statistical survival analyses. Elevated expression of each one of the two CHAC1 transcript variants is correlated with increased likelihood of tumour recurrence and decreased patient survival.

The mRNA expression of the two CHAC1 transcript variants may be determined by the methods of the invention by use of breast cancer tissue samples, such as those reviewed by a pathologist.

To determine the expression levels of CHAC1 transcript variants 1 and/or 2 in the practice of the present invention, any method known in the art may be utilized.

The present invention provides a more objective set of criteria, in the form of CHAC1 transcript variants 1 and/or 2 mRNA expression, to discriminate between breast cancer outcomes. In particularly preferred embodiments of the invention, the assays are used to discriminate between good and poor outcomes with respect to overall survival and distant disease free survival within 14 years after surgical intervention to remove breast cancer tumours. Comparisons that discriminate between outcomes after about 60, or about 120 months may also be performed.

While good and poor survival outcomes may be defined relatively in comparison to each other, a "good" outcome may be viewed as a better than 50 % overall survival rate after about 176 months post surgical intervention to remove breast cancer tumour(s). A "good" outcome may also be a better than about 50 % distant disease free survival after about 168 months post surgical intervention. A "poor" outcome may be viewed as a 50 % or less overall survival rate after about 112 months post surgical intervention to remove breast cancer tumour(s). A "poor" outcome may also be about a 50 % distant disease free survival after about 67 months post surgical intervention.

In one embodiment of the invention, the isolation and analysis of a breast cancer tissue sample may be performed as follows:
(1) Breast cancer tumour specimens are obtained immediately after surgery, and a part of the tissue is pulverized under cooling with liquid nitrogen and stored at -70 °C.
(2) RNA is extracted from the tumour tissue.
(3) RNA is purified, and Reverse transcription is performed.
(4) Q-PCR is performed using transcript variant specific oligonucleotides for CHAC1 transcript variants:
   Variant 1:
      Forward: 5'-ATGCCTGGCCGTGTGG-3' (SEQ ID NO 1),
      Reverse: 5'- GCTTACCTGCTCCCCTTGC -3' (SEQ ID NO 2),
      TaqMan Probe: 5'-FAM-CAGCCCTCATGATCTTCAAGGAGCGT-TAMRA-3' (SEQ ID NO 3);
   Variant 2:
      Forward: 5'-GGTTCTGCTCCCCTTGCA-3' (SEQ ID NO 4),
      Reverse: 5'-CGTGTGGTGACGCTCCTTG-3' (SEQ ID NO 5),
      TaqMan Probe: 5'-FAM-CCCAAGTGCAGCCCTCATGA-TAMRA-3' (SEQ ID NO 6).

**Tab. 1 shows the oligonucleotide sequences and labelling**

| **SEQ ID No** | **Sequence (5' - 3')** | **Labelling** |
|---|---|---|
| 1 | ATGCCTGGCCGTGTGG | - |
| 2 | GCTTACCTGCTCCCCTTGC | - |
| 3 | CAGCCCTCATGATCTTCAAGGAGCGT | 5'-FAM + 3'-TAMRA |
| 4 | GGTTCTGCTCCCCTTGCA | - |
| 5 | CGTGTGGTGACGCTCCTTG | - |
| 6 | CCCAAGTGCAGCCCTCATGA | 5'-FAM + 3'-TAMRA |

With use of the present invention, skilled physicians may prescribe treatments based on prognosis determined via CHAC1 transcript variant 2 mRNA expression analysis in the solid tissue biopsy.

After the general description of the invention depicted above, the invention will be more readily understood through reference to the following example which is provided by way of illustration, and is not intended to be limiting of the present invention, unless specified.

### Example I

Clinical specimen collection and clinicopathological parameters.

CHAC1 transcript variants 1 and 2 as well as total CHAC1 mRNA-expression were measured in 106 breast tumour tissues. Tissue specimens were obtained immediately after surgery, were brought to our pathologist, and a part of the tissue was pulverized under cooling with liquid nitrogen and stored at -70 °C.

Total cellular RNA was extracted from the tumour specimens using the acid guanidium thiocyanate-phenol-chloroform method. Integrity was evaluated by assessing the 18S- and 28S-ribosomal RNA bands in 2 % ethidium bromide-stained agarose gel. RNA concentration was measured by spectrophotometric analysis.

Reverse transcription of RNA was performed in a final volume of 20 µl containing 1 x RT-Buffer (50mM Tris-HCl, pH 8.3, 75mM KC1, 5mM MgCl₂), 40 U of rRNasin RNase Inhibitor (Promega, Madison, WI, USA), 10mM dithiothreitol, 200U of M-MLV Reverse Transcriptase (Gibco BRL, Gaithersburg, MD, USA), 5 µM random hexamers (Applied Biosystems, Foster City, CA, USA) and 800 ng of total RNA. The samples were first incubated at 65 °C for 5 min and then quick-chilled on ice. After adding the M-MLV enzyme, the samples were incubated at 25 °C for 10 min and at 37 °C for 60 min, followed by a period of 15 min at 70 °C to inactivate the reverse transcriptase enzyme.

Primers and probe for Q-PCR for CHAC1 were purchased from Applied Biosystems (Applied Biosystems Assay ID: Hs00899499_g1).

Primers and probe for CHAC1 transcript variants: Transcript variant 1: Forward: 5'-ATGCCTGGCCGTGTGG-3', Reverse: 5'-GCTTACCTGCTCCCCTTGC-3', TaqMan Probe: 5'-FAM-CAGCCCTCATGATCTTCAAGGAGCGT-TAMRA-3'; Transcript variant 2: Forward: 5'-GGTTCTGCTCCCCTTGCA-3', Reverse: 5'-CGTGTGGTGACGCTCCTTG-3', TaqMan Probe: 5'-FAM-CCCAAGTGCAGCCCTCATGA-TAMRA-3'.

Primers and probes for the TATA box-binding protein (TBP; used as endogenous RNA control) were used according to Bieche et al. All reactions were checked if they are specific for mRNA and do not amplify genomic DNA.

PCR reactions were performed using an ABI Prism 7900HT Detection System (Applied Biosystems, Foster City, CA, USA) with a total volume of 25 µl reaction mixture containing 5 µl of each appropriately diluted RT sample (concentration of the originally RNA in the PCR reaction is 2ng/µl), 12.5 µl TaqMan Universal PCR Master Mix (Applied Biosystems, Foster City, CA, USA), 900 nM of each primer and 250 nM of the probe. The thermal cycling conditions comprised an initial incubation at 50 °C for 2 min, a denaturing step at 95 °C for 10 min and 40 cycles at 95 °C for 15 s and at 65 °C for 1 min.

The characteristics of the 106 breast cancer patients and the clinicopathological features of the tumour specimens are shown in Table 2.

**Table 2**

| | | | **CHAC1 mRNA expression logarithmic values (norm. to TBP)** | |
|---|---|---|---|---|
| | | n | Mean (+/- SD) | *P* |
| | | | | |
| **Size** | T1 | 33 | -0.41 (0.59) | **0.011** |
| | T2/3/4 | 73 | -0.01 (0.82) | |
| | | | | |
| **LN** | negative | 44 | -0.17 (0.72) | n.s. |
| | positive | 56 | -0.15 (0.80) | |
| | n.a. | 6 | | |
| | | | | |
| **Tumor grade** | I | 26 | -0.41 (0.76) | **0.004** |
| | II | 58 | -0.14 (0.67) | |
| | III | 20 | 0.35 (0.93) | |
| | n.a. | 2 | | |
| | | | | |
| **MP** | premenopausal | 20 | -0.35 (0.76) | n.s. |
| | postmenopausal | 86 | -0.07 (0.78) | |
| | | | | |
| **ER** | neg | 37 | 0.38 (0.73) | **< 0.001** |
| | pos | 69 | -0.39 (0.67) | |
| | | | | |
| **PR** | neg | 41 | 0.40 (0.76) | **< 0.001** |
| | pos | 65 | -0.45 (0.59) | |
| | | | | |
| **HR** | neg | 34 | 0.46 (0.68) | **< 0.001** |
| | pos | 72 | -0.39 (0.67) | |
| | | | | |
| **HER2** | score 0/+ | 53 | 0,01 (0.89) | n.s. |
| | score ++/+++ | 34 | -0.22 (0.63) | |
| | n.a. | 19 | | |

| | | | | |
|---|---|---|---|---|
| Abbreviations: LN, lymph node status; MP, menopausal status; ER, estrogen receptor status; PR, progesterone receptor status; HR, hormon receptor status; n.a, not available. | | | | |

The prognostic significance of CHAC1 was examined in breast cancer. Total CHAC1 mRNA-expression, CHAC1 transcript variants 1 and CHAC1 transcript variants 2 were classified into low and high using the respective median expression values as cut-off. Univariate analysis of all 106 breast cancer patients revealed no significant association of total CHAC1 mRNA-expression with clinical outcome. In contrast to this, observation transcript-variant 2 was associated with poor outcome for OS (p = 0.017; Table 3) and with a poor DDFS (p = 0.009) (Table 3). The Kaplan Meier Survival Curves for transcript-variant 2 are shown in Figure 1.

**Table 3**

| | | **OVERALL SURVIVAL** | | | **DISTANT DISEASE FREE SURVIVAL** | | |
|---|---|---|---|---|---|---|---|
| **Variable** | | **No.Patients** | | ***P*** | **No**.**Patients** | | *P* |
| | | **(died/total)** | | **(logrank-Test)** | **(relapsed/total)** | | **(logrank-Test)** |
| | | | | | | | |
| **Size** | T1 | | 8/33 | **0.037** | | 9/33 | **0.031** |
| | T2/3/4 | | 39/73 | | | 40/73 | |
| **LN** | negative | | 13/44 | **0.016** | | 14/44 | **0.008** |
| | positive | | 31/56 | | | 32/56 | |
| **Tumor grade** | grade I | | 14/26 | n s | | 15/26 | n s |
| | grade II | | 26/58 | | | 27/58 | |
| | grade III | | 7/20 | | | 7/20 | |
| **MP** | premenopausal | | 5/20 | **0.039** | | 5/20 | n s |
| | postmenopausal | | 42/86 | | | 44/86 | |
| **HER2** | neg | | 25/53 | n s | | 26/53 | n s |
| | pos | | 11/34 | | | 12/34 | |
| **ER** | neg | | 15/37 | n s | | 16/37 | n s |
| | pos | | 32/69 | | | 33/69 | |
| **PR** | neg | | 18/41 | n s | | 19/41 | n s |
| | pos | | 29/65 | | | 30/65 | |
| **HR** | neg | | 14/34 | n s | | 15/34 | n s |
| | pos | | 33/72 | | | 34/72 | |
| **Chemotherapy** | no | | 24/56 | n s | | 25/56 | n s |
| | yes | | 23/50 | | | 24/50 | |
| **Radiation therapy** | no | | 18/36 | n s | | 19/36 | n s |
| | yes | | 29/69 | | | 30/69 | |
| **Endocrine therapy** | no | | 17/41 | n s | | 18/41 | n s |
| | yes | | 30/65 | | | 31/65 | |
| **CHAC1 mRNA expression** | low (≤ median) | | 20/53 | n s | | 21/53 | n s |
| | high (> median) | | 27/53 | | | 28/53 | |
| | | | | | | | |
| **CHAC1 transcript-variant 1** | low (≤ median) | | 19/53 | n s | | 21/53 | n s |
| **mRNA expression** | high (> median) | | 28/53 | | | 28/53 | |
| | | | | | | | |
| **CHAC1 transcript-variant 2** | low (≤ median) | | 19/53 | **0.017** | | 20/53 | **0.009** |
| **mRNA expression** | high (> median) | | 28/53 | | | 29/53 | |

| | | | | | | | |
|---|---|---|---|---|---|---|---|
| Abbreviations LN, lymph node status, MP, menopausal status, HR, hormone receptor status, | | | | | | | |

When adjusting for clinico-patholocical factors and therapies, the multivariate survival analysis showed significant prognostic value of transcript-variant 1 [RR_{death} 6.7 (2.4 - 18.9); p<0.001), RR relapse or death 5.3 (1.9 - 14.3); p=0.001] and transcript-variant 2 [RR_{death} 4.9 (2.0 - 12.4); p < 0.001), RR_{relapse or dearth} 5.1 (2.0 -12.9); p < 0.001] (Table 4).

**Table 4**

| **A** | | | | | | |
|---|---|---|---|---|---|---|
| | | | **OVERALL SURVIVAL** | | | |
| | **Variable** | | **RR o. death** | | **RR o. death** | |
| | | | (95% Cl)^{*1} | **P** | (95% Cl)^{*2} | **P** |
| | | | | | | |
| | **Age** | ≤ median age | **2.9(1.1-8.0)** | **0.04** | **3.6(1.4-9.7)** | **0.01** |
| | | > median age | | | | |
| | | | | | | |
| | **Size** | T1 | **3.7(1.2-11.0)** | **0.02** | **3.2(1.1-9.3)** | **0.03** |
| | | T2/3/4 | | | | |
| | | | | | | |
| | **LN** | negative | 25(09-67) | 007 | 20(08-52) | 016 |
| | | positive | | | | |
| | | | | | | |
| | **Tumor grade** | grade I | 10(06-18) | 094 | 10(06-17) | 091 |
| | | grade II | | | | |
| | | grade III | | | | |
| | | | | | | |
| | **MP** | premenopausal | 14(04-47) | 061 | 07(02-25) | 062 |
| | | postmenopausal | | | | |
| | | | | | | |
| | **HER2** | neg | 13(05-31) | 058 | 13(05-29) | 058 |
| | | pos | | | | |
| | | | | | | |
| | **HR** | neg | 130(23-732) | 000 | 42(10-173) | 004 |
| | | pos | | | | |
| | | | | | | |
| | **Chemotherapy** | no | 05(02-13) | 015 | 05(02-14) | 021 |
| | | yes | | | | |
| | | | | | | |
| | **Radiation therapy** | no | 09(04-22) | 089 | 12(05-29) | 060 |
| | | yes | | | | |
| | | | | | | |
| | **Endocrine therapy** | no | 04(01-13) | 013 | 09(03-29) | 082 |
| | | yes | | | | |
| | | | | | | |
| | **CHAC1 mRNA expression*** | low (≤ median) | **6.7(2.4-18.9)** | **<0.001** | **4.9(2.0-12.4)** | **<0.001** |
| | | high (> median) | | | | |

| **B** | | | | | | |
|---|---|---|---|---|---|---|
| | | | **DISTANT DISEASE FREE SURVIVAL** | | | |
| | | | **RR o. relapse or** | | **RR o. relapse or** | |
| | **Variable** | | **death (95% Cl)^{*1}** | **P** | **death (95% Cl)^{*2}** | **P** |
| | | | | | | |
| | **Age** | ≤ median age | **2.4(0.9-6.4)** | 007 | **3.0(1.2-7.6)** | **0.02** |
| | | > median age | | | | |
| | | | | | | |
| | **Size** | T1 | 27(10-75) | 005 | 26(10-71) | 005 |
| | | T2/3/4 | | | | |
| | | | | | | |
| | **LN** | negative | 22(09-56) | 009 | 20(08-50) | 014 |
| | | positive | | | | |
| | | | | | | |
| | **Tumor grade** | grade I | 09(05-15) | 068 | 08(05-15) | 055 |
| | | grade **II** | | | | |
| | | grade III | | | | |
| | | | | | | |
| | **MP** | premenopausal | 15(04-48) | 054 | 08(03-27) | 076 |
| | | postmenopausal | | | | |
| | | | | | | |
| | **HER2** | neg | 09(04-22) | 090 | 10(04-21) | 091 |
| | | pos | | | | |
| | | | | | | |
| | **HR** | neg | 71(15-346) | **0.01** | 31(08-116) | 009 |
| | | pos | | | | |
| | | | | | | |
| | **Chemotherapy** | no | 07(03-19) | 050 | 07(03-19) | 053 |
| | | yes | | | | |
| | | | | | | |
| | **Radiation therapy** | no | 10(04-23) | 099 | 12(05-28) | 067 |
| | | yes | | | | |
| | | | | | | |
| | **Endocrine therapy** | no | 05(01-17) | 026 | 10(03-32) | 097 |
| | | yes | | | | |
| | | | | | | |
| | **CHAC1 mRNA expression*** | low (≤median) | **5.3(1.9-14.3)** | **0.001** | **5.1(2.0-12.9)** | **<0.001** |
| | | high (> median) | | | | |

| | | | | | | |
|---|---|---|---|---|---|---|
| Abbreviations LN, lymph node status, MP, menopausal status, HER2, human epidermal growth factor receptor 2 status, HR, hormon receptor status, * 1) CHAC1 transcript-variant 1 included * 2) CHAC1 transcript-variant 2 included | | | | | | |

### References:

1. Ferlay J, Shin HR, Bray F, Forman D, Mathers C, Parkin DM. Estimates of worldwide burden of cancer in 2008: GLOBOCAN 2008. Int J Cancer 2010; Epub ahead of print; PMID: 20560135
2. NCI's Surveillance, Epidemiology, and End Results Program (SEER) publication SEER Cancer Statistics Review 1973-1997.
3. Goldhirsch A, Glick JH, Gelber RD, Coates AS, Senn HJ. Meeting highlights: International Consensus Panel on the Treatment of Primary Breast Cancer. Seventh International Conference on Adjuvant Therapy of Primary Breast Cancer. J Clin Oncol 2001;19:3817-27.
4. Mark HF, Aswad B, Bassily N, Taylor W, Brown S, Sun CL, Samy M, Zolnierz K, Wong E, Bland KI, Hsu PH. HER-2/neu gene amplification in stages I-IV breast cancer detected by fluorescent in situ hybridization. Genet Med. 1999;1:98-103.
5. Fernandez PM, Tabbara SO, Jacobs LK, Manning FCR, Tsangaris TN, Schwartz AM, Kennedy KA, Patierno SR. Overexpression of the glucose-regulated stress gene GRP78 in malignant but not benign human breast lesions. Breast Cancer Res Treat 2000. 59: 15-26.
6. Scriven P, Coulson S, Haines R, Balasubramanian S, Cross S, Wyld L. Activation and clinical significance of the unfolded protein response in breast cancer. Br J Cancer. 2009;101(10):1692-8.
7. Gargalovic P, Imura M, Zhang B, et al. Identification of inflammatory gene modules based on variations of human endothelial cell responses to oxidized lipids. Proc Natl Acad Sci U S A 2006;103:12741-6.
8. Le QT, Denko NC, Giaccia AJ. Hypoxic gene expression and metastasis. Cancer Metastasis Rev 2004;23:293-310.
9. Scriven P, Brown NJ, Pockley AG, Wyld L. The unfolded protein response and cancer: a brighter future unfolding? J Mol Med 2007;85:331-41.
10. Bieche I, Onody P, Laurendeau I, et al. Real-time reverse transcription-PCR assay for future management of ERBB2-based clinical applications. Clin Chem 1999;45:1148-56.

### Supplementary Material and Methods:

### Patients and samples:

The study was approved by the local Institutional Ethics Review Board and it was performed in concordance with the Reporting Recommendations for Tumour Marker Prognostic Studies of the National Cancer Institute (REMARK) and the Declaration of Helsinki. Clinical, pathological and follow-up data were stored in a database in accordance with our hospital privacy rules. The patients were treated at the Department of Obstetrics and Gynaecology of the Innsbruck Medical University, Austria between April 1990 and March 2004.

Frozen breast tissue samples from 106 patients with breast cancer [Median age at diagnosis was 60.4 years (35.5 to 89.7)] and ten patients with benign neoplastic breast diseases [Median age at diagnosis was 40.1 (27.3 to 66.9)] were analyzed. The median observation period of all patients was 7.6 years (0.9 to 17).

None of the patients was diagnosed to have a distant metastatic disease at primary surgery. All patients were monitored within the outpatient follow-up program of the Department of Obstetrics and Gynaecology, Innsbruck Medical University. Follow-up information was available for all patients.

No neoadjuvant chemotherapy was applied to the patients included in the study.

23 % of the breast cancer patients received only chemotherapy (n = 24) and 37 % only endocrine therapy (n = 39). 25 % of the study population received chemotherapy and endocrine therapy (n = 26). 6 % of the breast cancer patients received no adjuvant therapy (n = 6) and 10 % patients only radiation therapy (n = 11). Radiation therapy was applied in combination with chemotherapy or endocrine therapy in 55 % (n = 58). None of the patients received Anti-HER2 therapy. Tumour specimens were obtained immediately after surgery, were brought to our pathologist, and a part of the tissue was pulverized under cooling with liquid nitrogen and stored at -70 °C. ER and Progesterone receptor (PR) status were identified immunohistochemically.

### RNA isolation and Reverse transcription.

Total cellular RNA was extracted from the tumour specimens using the acid guanidium thiocyanate-phenol-chloroform method. Integrity was evaluated by assessing the 18S- and 28S-ribosomal RNA bands in 2 % ethidium bromide-stained agarose gel. RNA concentration was measured by spectrophotometric analysis.

Reverse transcription of RNA was performed in a final volume of 20 µl containing 1 x RT-Buffer (50mM Tris-HCl, pH 8.3, 75mM KCl, 5mM MgCl2), 40U of rRNasin RNase Inhibitor (Promega, Madison, WI, USA), 10mM dithiothreitol, 200U of M-MLV Reverse Transcriptase (Gibco BRL, Gaithersburg, MD, USA), 5 µM random hexamers (Applied Biosystems, Foster City, CA, USA) and 800 ng of total RNA. The samples were first incubated at 65 °C for 5 min and then quick-chilled on ice. After adding the M-MLV enzyme, the samples were incubated at 25 °C for 10 min and at 37 °C for 60 min, followed by a period of 15 min at 70 °C to inactivate the reverse transcriptase enzyme.

### Primers and probes:

Primers and probe for Q-PCR for CHAC1 were purchased from Applied Biosystems (Applied Biosystems Assay ID: Hs00899499_g1).

Primers and probe for CHAC1 transcript variants: Transcript variant 1: Forward: 5'-ATGCCTGGCCGTGTGG-3', Reverse: 5'-GCTTACCTGCTCCCCTTGC-3', TaqMan Probe: 5'-FAM-CAGCCCTCATGATCTTCAAGGAGCGT-TAMRA-3'; Transcript variant 2: Forward: 5'-GGTTCTGCTCCCCTTGCA-3', Reverse: 5'-CGTGTGGTGACGCTCCTTG-3', TaqMan Probe: 5'-FAM-CCCAAGTGCAGCCCTCATGA-TAMRA-3'.

Primers and probes for the TATA box-binding protein (TBP; used as endogenous RNA control) were used according to Bieche et al (10). All reactions were checked if they are specific for mRNA and do not amplify genomic DNA.

### Real-time PCR amplification:

PCR reactions were performed using an ABI Prism 7900HT Detection System (Applied Biosystems, Foster City, CA, USA) with a total volume of 25 µl reaction mixture containing 5 µl of each appropriately diluted RT sample (concentration of the originally RNA in the PCR reaction is 2ng/µl), 12.5 µl TaqMan Universal PCR Master Mix (Applied Biosystems, Foster City, CA, USA), 900 nM of each primer and 250 nM of the probe. The thermal cycling conditions comprised an initial incubation at 50 °C for 2 min, a denaturing step at 95 °C for 10 min and 40 cycles at 95 °C for 15 s and at 65 °C for 1 min.

### Statistical Analyses:

SPSS 18.0, STATA/MP 10.0 and BayesX 1.51 were used for the statistical analyses.

For survival analysis the CHAC1 mRNA-expression was dichotomized into low and high using the median expression value. Distant disease-free survival (DDFS) was defined as the time from surgery to histopathological confirmation of distant metastases or death. Overall survival (OS) was defined as the time from surgery to death from any cause or to the last clinical inspection. To estimate hazard ratios with 95 % confidence-intervals (95 % CIs), we first calculated univariate Kaplan-Meier curves for dichotomized age, tumour-size, stage, grade, menopausal-status, oestrogen, progesterone and HER2-status, chemotherapy and CHAC1 mRNA-expression. Additionally the application of endocrine therapy and radiation therapy was considered for breast cancer specimens. Kaplan Meier Curves and the log-rank test were used to compare the survival distributions between groups.

Secondly a time-independent Cox proportional hazard model was calculated including the significant variables of the univariate survival models. Dichotomized CHAC1 transcript variant 1 and variant 2 mRNA-expression values were used as exposure variables. P-values less than 0.05 were considered as statistically significant.

## Claims

1. An assay to determine the amount of CHAC1 transcript present in a sample **characterized in** the use of oligonucleotides, capable of hybridizing to mRNA of CHAC1 transcript variant 1 or CHAC1 transcript variant 2.

2. An assay according to claim 1, **characterized in that** the oligonucleotides comprise a sequence of nucleotides from the group consisting of SEQ ID NOS 1, 2, 3, 4, 5, and 6.

3. A method to determine the survival outcome of a breast cancer patient or to determine prognosis of a breast cancer patient, **characterized in that** the method comprises the analysis of breast cancer cell samples of the breast cancer patient for the expression level of CHAC1 transcript variants 1 and/or 2.

4. Method according to claim 3, **characterized in that** overexpression of CHAC1 transcript variant 1 and/or 2 is assayed.

5. Method according to claim 3 or claim 4, **characterized in that** assaying comprises preparing RNA, preferably mRNA, from said sample.

6. Method according to claim 5, **characterized in that** the RNA is used for quantitative PCR.

7. Method according to any of claims 3 to 6, **characterized in that** the sample is a breast cancer tissue sample.

8. A method to determine therapeutic treatment for a breast cancer patient based upon said patient's expected survival, **characterized in that** the method comprises determining a survival outcome for said patient by analyzing a sample of breast cancer cells from said patient for the mRNA expression level of CHAC1 transcript variants 1 and/or 2 and selecting the appropriate treatment for a patient with such a survival outcome.

9. A method to determine the expression level of CHAC1 transcript variants 1 and/or 2 present in a sample of breast cancer patient of a subject by measuring the amount of the mRNA present in the breast cancer cells.

10. The method according to claim 9, **characterized in that** the mRNA expression level of CHAC1 transcript variants 1 and/or 2 is measured by quantitative PCR.

11. An oligonucleotide selected from the group SEQ ID NOS: 1, 2, 3, 4, 5, and 6.
